# EUROPEAN PATENT APPLICATION

(11) **EP 4 674 946 A1**
(43) Date of publication of application: **07.01.2026**
(21) Application number: 24875618.1
(22) Date of filing: 24.10.2024
(51) Int. Cl.: C12N 1/19, C12N 9/10, C12N 15/54

(54) **NOVEL GENETIC RECOMBINATION MARKER AND USE THEREOF**

(30) Priority: 22.05.2024 WO PCT/JP2024/018869
(71) Applicant: KAO CORPORATION, Tokyo 103-8210 (JP)
(72) Inventor: KANEDA, Jitsuro, Tokyo 103-8210 (JP); TOHATA, Masatoshi, Tokyo 103-8210 (JP); TERAI, Mika, Tokyo 103-8210 (JP); NONAKA, Kyoshiro, Tokyo 103-8210 (JP); TAKAHASHI, Fumikazu, Tokyo 103-8210 (JP)
(74) Representative: Jones, Nicholas Andrew
(86) International application number: PCT/JP2024/037995
(87) International publication number: WO 2025/243561

(57) **Abstract**

Provided are a novel marker for gene recombination of *Starmerella bombicola* and a method for using the same. The present invention provides an adenine auxotrophic selection marker consisting of a gene encoding a phosphoribosyl-glycinamide formyltransferase-like protein or a gene equivalent thereto. The present invention also provides a *Starmerella bombicola* mutant strain in which expression of a phosphoribosyl-glycinamide formyltransferase-like protein or a protein equivalent thereto is suppressed or the protein is inactivated.

## Description

### Field of the Invention

The present invention relates to a novel marker for gene recombination and use thereof.

### Background of the Invention

Sophorolipid is a glycolipid which is composed of sophorose linked to a long-chain hydroxy fatty acid, and produced by microorganisms, mainly, yeasts. Sophorolipid is an amphipathic lipid having strong surface activity and excellent biodegradability. Therefore, its application as a biosurfactant has received attention in recent years. Sophorolipid has good skin affinity because of being a microbic product and being composed mainly of a nonionic component, and as such, is used as a permeation enhancer for cosmetics. Furthermore, sophorolipid is excellent in biodegradability and effective even when added in a small amount, and as such, is increasingly used in the field of cleansing agents such as dishwashing detergents.

Improvement in productivity of sophorolipid by microorganisms requires enhanced activity of the microorganisms. Approaches for such enhanced activity mainly include gene recombination techniques. In gene recombination in microorganisms, selection markers such as drug resistance genes or auxotrophy-related genes are often used, and genetically modified microorganisms of interest are selected with these markers by using, as an index, drug resistance or an auxotrophic trait that has been lost or acquired. However, the genetically modified microorganisms into which a foreign gene not intrinsic to the microorganisms is introduced have, for example, a risk that the introduced foreign gene might be horizontally transferred to other microorganisms and adversely affect the ecosystem.

Meanwhile, the enforcement regulation of the Act on the Conservation and Sustainable Use of Biological Diversity through Regulations on the Use of Living Modified Organisms (Cartagena Act) stipulates rules to exclude organisms that fall within self-cloning or natural occurrence from organisms subject to the Act even if the organisms are obtained through the use of gene recombination techniques. Thus, the organisms that fall within self-cloning or natural occurrence are not included in living modified organisms. Accordingly, even if gene recombination techniques are used for improvement in productivity of sophorolipid by microorganism, the microorganisms are desirably obtained through self-cloning or natural occurrence.

*Starmerella bombicola* [former *Candida bombicola*], a nonpathogenic basidiomycetous yeast, is well known as a yeast which produces sophorolipid. In the modification of *Starmerella bombicola,* foreign antibiotic resistance genes such as hygromycin resistance gene or nourseothricin resistance gene are often used as markers for selection of recombinants, and only ura3 gene is known as an example of an endogenous gene-derived selection marker necessary for self-cloning (endogenous selection marker, self-marker) (Non Patent Literature 1). Thus, a novel endogenous selection marker (self-marker) is demanded. In general, genes involved in the biosynthesis of amino acids, nucleic acids, or the like are considered as endogenous selection marker (self-marker) candidates. However, host strains functionally deficient in the genes which can be used in self-cloning are difficult to establish.

(Non Patent Literature 1) Inge N. A. Van Bogaert et al., Yeast, 2007, 24: 201-208

### Summary of the Invention

The present invention relates to the following 1) to 7).
1) A *Starmerella bombicola* mutant strain in which expression of a phosphoribosyl-glycinamide formyltransferase-like protein or a protein equivalent thereto is suppressed or the protein is inactivated.
2) A method for producing a *Starmerella bombicola* mutant strain, comprising suppressing expression of or inactivating a phosphoribosyl-glycinamide formyltransferase-like protein or a protein equivalent thereto in *Starmerella bombicola.*
3) A method for transforming *Starmerella bombicola,* comprising using the mutant strain according to 1) as a host.
4) A transformant obtained by the method according to 3).
5) A method for producing a glycolipid, comprising culturing the mutant strain according to 1) or the transformant according to 4).
6) An adenine auxotrophic selection marker consisting of a gene encoding a phosphoribosyl-glycinamide formyltransferase-like protein or a gene equivalent thereto.
7) A vector or a DNA fragment comprising a gene encoding a phosphoribosyl-glycinamide formyltransferase-like protein or a gene equivalent thereto.

### Brief Description of Drawing

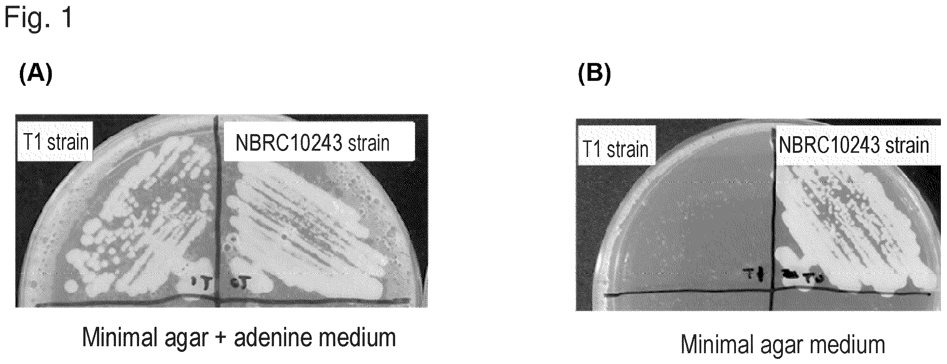

Fig. 1 illustrates the growth of a *Starmerella bombicola* mutant strain (T1 strain) having a mutation in a gene consisting of the nucleotide sequence of SEQ ID NO: 1 and its parent strain *Starmerella bombicola* NBRC10243 in (A) a minimal agar + adenine medium and (B) a minimal agar medium.

### Detailed Description of the Invention

If a novel endogenous selection marker (self-marker) for gene recombination which can be used in *Starmerella bombicola* and a gene recombination technique using the self-marker can be developed, these can serve as basic technology useful for production of a product including glycolipids using *Starmerella bombicola.*

The present invention relates to provision of a novel marker for gene recombination of *Starmerella bombicola* and a method for using the same.

The present inventors continued the subculture of a type strain of *Starmerella bombicola* used in glycolipid production and consequently successfully obtained a mutant strain which exhibited adenine auxotrophy as a result of introducing a mutation into a specific gene. The present inventors also found that: a transformation system of *Starmerella bombicola* with the mutant strain as a host can be constructed; and in this respect, a transformant can be efficiently selected by using the specific gene as an endogenous selection marker (self-marker), and using the presence or absence of adenine auxotrophy as an index.

The present invention provides a *Starmerella bombicola* mutant strain which exhibits adenine auxotrophy. Use of the *Starmerella bombicola* mutant strain of the present invention as a host enables *Starmerella bombicola* to be efficiently transformed by using the presence or absence of adenine auxotrophy as an index.

All patent and non-patent literatures and other publications cited herein are hereby incorporated by reference in their entirety.

### (1. Definition)

In the present specification, the identity of an amino acid sequence or a nucleotide sequence is calculated by the Lipman-Pearson method (Science, 1985, 227: 1435-1441). Specifically, the identity is calculated by analysis using the search homology program of genetic information processing software GENETYX Ver. 12 with the unit size to compare (ktup) set to 2.

In the present specification, the term "at least 90% identity" regarding an amino acid sequence or a nucleotide sequence refers to 90% or more, preferably 95% or more, more preferably 97% or more, further more preferably 98% or more, further more preferably 99% or more identity.

In the present specification, the term "amino acid sequence obtained by deletion, substitution, addition, or insertion of one or several amino acids" refers to an amino acid sequence obtained by deletion, substitution, addition, or insertion of 1 or more and 20 or less, preferably 1 or more and 10 or less, more preferably 1 or more and 8 or less, further more preferably 1 or more and 5 or less, even more preferably 1 or more and 3 or less amino acids. The term "nucleotide sequence obtained by deletion, substitution, addition, or insertion of one or several nucleotides" refers to a nucleotide sequence derived by the deletion, substitution, addition, or insertion of 1 or more and 60 or less, preferably 1 or more and 30 or less, more preferably 1 or more and 24 or less, further more preferably 1 or more and 15 or less, even more preferably 1 or more and 9 or less nucleotides. In the present specification, the "addition" of an amino acid or a nucleotide includes the addition of the amino acid or the nucleotide to one end and both ends of a sequence.

In the present specification, the terms "upstream" and "downstream" regarding a gene refer to upstream and downstream in the transcriptional direction of the gene. For example, the term "gene located downstream of a promoter" means that the gene resides on the 3' side of the promoter in a DNA sense strand, and the term "upstream of a gene" means a region on the 5' side of the gene in a DNA sense strand.

In the present specification, the term "operable linking" between a control region such as a promoter and a gene means that the gene and the control region are linked such that the gene is capable of being expressed under the control of the control region. Procedures for the "operable linking" between a gene and a control region are well known to those skilled in the art.

In the present specification, the term "original" used for a function, a property, or a trait of a cell is used for representing that the cell inherently has the function, the property, or the trait. By contrast, the term "foreign" is used for representing that the function, the property, or the trait is introduced from the outside but not inherent in the cell. For example, the "foreign" gene or polynucleotide is a gene or a polynucleotide introduced to a cell from the outside. The foreign gene or polynucleotide may be derived from the same species of organism as that of a cell into which it has been introduced, or may be derived from a different species of organism therefrom (i.e., a heterologous gene or polynucleotide).

In the present specification, the term "adenine auxotrophy" means that cell growth requires adenine to be supplied from the outside. An adenine auxotrophic microbe strain cannot substantially grow in an adenine-free medium but can grow in an adenine-containing medium. The adenine auxotrophic microorganism strain cannot grow in a minimal solid medium without adenine and on the other hand, can grow in a minimal solid medium containing adenine, for example, under conditions shown in Examples described later.

In the present specification, the term "self-cloning" refers to the case where a donor of insert DNA in a recombinant and a host belong to the same species as to organisms obtained through the use of gene recombination techniques. In the present specification, the term "natural occurrence" refers to the case where a donor of insert DNA in a recombinant and a host are classified into different species, genetic exchange has been found to occur therebetween in nature and the donor of insert DNA in a recombinant and the host belong to both of these species, among organisms obtained through the use of gene recombination techniques. In the present specification, the term "mutation" refers to change in genetic information and is caused by a mistake in DNA replication, DNA damage ascribable to a chemical, DNA or chromosomal damage ascribable to a replication mistake or radiation, gene disruption ascribable to transposition of transposons, or the like. A mutation which occurs independently from artificial manipulation is referred to as "spontaneous mutation".

### (2. Starmerella bombicola mutant strain)

The present inventors continued the subculture of a type strain of *Starmerella bombicola* used in glycolipid production and consequently obtained a mutant strain which exhibited adenine auxotrophy by introducing a mutation into a gene encoding a polypeptide consisting of the amino acid sequence of SEQ ID NO: 2. The amino acid sequence of SEQ ID NO: 2 is an amino acid sequence based on specific ORF found as a result of conducting ORF analysis by the present inventors on *Starmerella bombicola* which has not previously been sufficiently studied by ORF analysis. As shown in Examples described later, the *Starmerella bombicola* mutant strain having a mutation in a gene encoding a polypeptide consisting of the amino acid sequence of SEQ ID NO: 2 can grow in an adenine-containing medium and on the other hand, cannot grow in an adenine-free medium. Accordingly, the polypeptide consisting of the amino acid sequence of SEQ ID NO: 2 is presumed to be a polypeptide involved in an adenine biosynthesis pathway of *Starmerella bombicola,* and is probably a polypeptide having phosphoribosyl-glycinamide formyltransferase activity because of having an FMT_core_GART domain as a result of CD search of NCBI. Accordingly, the polypeptide consisting of the amino acid sequence of SEQ ID NO: 2 has been designated as a phosphoribosyl-glycinamide formyltransferase-like protein of *Starmerella bombicola.* When expression of the phosphoribosyl-glycinamide formyltransferase-like protein is suppressed or the protein is inactivated by reduction or elimination of the protein function, adenine non-auxotrophic *Starmerella bombicola* has defective adenine biosynthesis or adenine biosynthesis incapability so that adenine auxotrophy is imparted to the *Starmerella bombicola.* On the contrary, when the phosphoribosyl-glycinamide formyltransferase-like protein is normally expressed in *Starmerella bombicola* having adenine auxotrophy due to suppressed expression of the phosphoribosyl-glycinamide formyltransferase-like protein or inactivation of the protein by reduction or elimination of the protein function, the adenine auxotrophy of the *Starmerella bombicola* can be canceled. Specifically, the phosphoribosyl-glycinamide formyltransferase-like protein of *Starmerella bombicola* is a protein having the ability to cancel adenine auxotrophy of adenine auxotrophic *Starmerella bombicola* and in other words, is a protein having a function of rendering adenine auxotrophic *Starmerella bombicola* adenine non-auxotrophic.

The present invention provides a *Starmerella bombicola* mutant strain (hereinafter, referred to as the mutant strain of the present invention). In the mutant strain of the present invention, expression of a phosphoribosyl-glycinamide formyltransferase-like protein or a protein equivalent thereto is suppressed or the protein is inactivated. The mutant strain of the present invention can be produced by suppressing expression of or inactivating the phosphoribosyl-glycinamide formyltransferase-like protein or the protein equivalent thereto in *Starmerella bombicola.* The mutant strain of the present invention exhibits adenine auxotrophy by suppressed expression or inactivation of the phosphoribosyl-glycinamide formyltransferase-like protein or the protein equivalent thereto.

In this context, the "phosphoribosyl-glycinamide formyltransferase-like protein" is a phosphoribosyl-glycinamide formyltransferase-like protein of *Starmerella bombicola* and is preferably a polypeptide consisting of the amino acid sequence of SEQ ID NO: 2.

The "protein equivalent to the phosphoribosyl-glycinamide formyltransferase-like protein" is a protein having the same function as that of the phosphoribosyl-glycinamide formyltransferase-like protein of *Starmerella bombicola* and includes a homolog or an ortholog of the phosphoribosyl-glycinamide formyltransferase-like protein of *Starmerella bombicola,* or a mutant thereof. The protein equivalent to the phosphoribosyl-glycinamide formyltransferase-like protein is preferably a polypeptide consisting of an amino acid sequence having at least 90% identity to the amino acid sequence of SEQ ID NO: 2. Examples of the amino acid sequence having at least 90% identity to the amino acid sequence of SEQ ID NO: 2 include an amino acid sequence obtained by deletion, substitution, addition, or insertion of one or several amino acids in the amino acid sequence of SEQ ID NO: 2.

Preferably, the mutant strain of the present invention is a mutant strain in which expression of the phosphoribosyl-glycinamide formyltransferase-like protein or the protein equivalent thereto is suppressed as compared with a strain before mutation (parent strain). In one embodiment, the mutant strain of the present invention can be a mutant strain in which an expression level of the phosphoribosyl-glycinamide formyltransferase-like protein or the protein equivalent thereto is decreased to 50% or less, preferably 40% or less, more preferably 30% or less, further more preferably 20% or less, even more preferably 10% or less, further preferably 5% or less as compared with the parent strain. Still further preferably, the mutant strain of the present invention can be a mutant strain in which an expression level of the phosphoribosyl-glycinamide formyltransferase-like protein or the protein equivalent thereto is lost to an undetectable level (equal to or less than a negative control or background expression level). The expression level of a protein or a polypeptide can be measured by a protein expression quantification method usually used, for example, but not limited to, a colorimetry method, a fluorescent method, Western blotting, ELISA, or radioimmunoassay.

Examples of the approach of suppressing expression of or inactivating the phosphoribosyl-glycinamide formyltransferase-like protein or the protein equivalent thereto in *Starmerella bombicola* include a method of deleting or inactivating a gene encoding the protein, a method of inactivating mRNA transcribed from a gene encoding the protein, a method of suppressing translation of mRNA of a gene encoding the protein by RNA interference or the like using siRNA, a method of mutating a gene encoding the protein to reduce activity of the protein, and a method of inactivating the protein by using an inhibitory substance such as an aptamer or an antibody. In a preferred embodiment, the mutant strain of the present invention is a mutant strain in which a gene encoding the phosphoribosyl-glycinamide formyltransferase-like protein or a gene equivalent thereto is deleted or inactivated.

In this context, the "gene encoding the phosphoribosyl-glycinamide formyltransferase-like protein" is a gene encoding the phosphoribosyl-glycinamide formyltransferase-like protein of *Starmerella bombicola* and is preferably a gene consisting of the nucleotide sequence of SEQ ID NO: 1.

The "gene equivalent to the gene encoding the phosphoribosyl-glycinamide formyltransferase-like protein" is a gene encoding the protein equivalent to the phosphoribosyl-glycinamide formyltransferase-like protein of *Starmerella bombicola* described above, and is preferably a gene consisting of a nucleotide sequence having at least 90% identity to the nucleotide sequence of SEQ ID NO: 1, more preferably a gene which consists of a nucleotide sequence having at least 90% identity to the nucleotide sequence of SEQ ID NO: 1, and encodes a polypeptide consisting of the amino acid sequence of SEQ ID NO: 2 or a polypeptide consisting of an amino acid sequence having at least 90% identity to the amino acid sequence of SEQ ID NO: 2. Examples of the nucleotide sequence having at least 90% identity to the nucleotide sequence of SEQ ID NO: 1 include a nucleotide sequence obtained by deletion, substitution, addition, or insertion of one or several nucleotides in the nucleotide sequence of SEQ ID NO: 1.

Examples of the approach of deleting or inactivating the gene of *Starmerella bombicola* include mutagenesis (deletion, insertion, substitution, or addition) of one or more nucleotides on the nucleotide sequence of the target gene, substitution of the nucleotide sequence with another nucleotide sequence, insertion of another nucleotide sequence to the nucleotide sequence, and partial or whole deletion of the nucleotide sequence. Alternatively, similar mutagenesis or substitution, insertion, or deletion of a nucleotide sequence may be performed for a control region such as a promoter region of the target gene. For example, the promoter activity of a promoter which controls the expression of the target gene is reduced or eliminated by mutagenesis or replacement with a low-expression promoter so that transcription of mRNA from the target gene can be reduced or eliminated to inactivate the target gene.

A method for genetic engineering of a microorganism known in the art can be used as a specific approach for the mutagenesis or substitution, insertion, or deletion of a nucleotide sequence. Examples of the method can include, but are not limited to, ultraviolet irradiation, site-directed mutagenesis, homologous recombination using SOE-PCR (splicing by overlap extension PCR: Gene, 1989, 77: 61-68), and genome editing using artificial DNA nuclease or programmable nuclease.

After the mutagenesis or substitution, insertion, or deletion of a nucleotide sequence, the mutant strain of the present invention can be obtained by selecting a cell having the desired mutation by gene analysis or evaluation of an expression level of mRNA or a polypeptide encoded by the target gene.

Alternatively, when the approach of deleting or inactivating the gene is homologous recombination using SOE-PCR, a mutant strain lacking the target gene can be obtained by incorporating a drug resistance marker gene into a DNA fragment for gene deletion with which DNA of the target gene is substituted, culturing cells into which the DNA fragment for deletion has been introduced in a medium containing a drug, and separating a grown colony. Further, the mutation may be confirmed by the gene analysis or the evaluation of a polypeptide expression level mentioned above. These procedures can produce the mutant strain of the present invention in which the gene encoding the phosphoribosyl-glycinamide formyltransferase-like protein or the gene equivalent thereto is deleted or inactivated.

Alternatively, the mutant strain of the present invention in which expression of the phosphoribosyl-glycinamide formyltransferase-like protein or the protein equivalent thereto is suppressed or the protein is inactivated may be obtained by confirming adenine auxotrophy in the mutant strain prepared by the procedures described above.

The mutant strain of the present invention may be a spontaneous mutant strain in which expression of the phosphoribosyl-glycinamide formyltransferase-like protein or the protein equivalent thereto is suppressed or the protein is inactivated by spontaneous mutation which occurs independently from artificial manipulation of mutation. The spontaneous mutant strain can be obtained, for example, by subculturing the parent strain by a culture method known in the art involving no artificial manipulation of mutation, subjecting cultured cells differing in trait from ordinary cells to gene analysis or evaluation of an expression level of mRNA or a polypeptide encoded by the target gene, and thereby selecting cells having the desired mutation, or confirming adenine auxotrophy.

Preferred examples of the mutant strain of the present invention include a *Starmerella bombicola* T1 strain (hereinafter, simply referred to as a T1 strain). The T1 strain was deposited with National Institute of Technology and Evaluation, Patent Microorganisms Depositary (#122, 2-5-8 Kazusakamatari, Kisarazu-shi, Chiba, Japan) on March 7, 2024 (accession No. NITE BP-04096). The T1 strain is a strain produced by subculturing a *Starmerella bombicola* NBRC10243 strain as a parent strain, as shown in Examples described later, and exhibits adenine auxotrophy as a result of a mutation in a gene encoding the phosphoribosyl-glycinamide formyltransferase-like protein. The T1 strain is a strain produced by subculture involving no artificial manipulation of mutation and is therefore considered to fall within a strain obtained by spontaneous mutation.

### (3. Transformation method)

The mutant strain of the present invention exhibits adenine auxotrophy by suppressed expression or inactivation of the phosphoribosyl-glycinamide formyltransferase-like protein or the protein equivalent thereto. The gene encoding the phosphoribosyl-glycinamide formyltransferase-like protein or the gene equivalent thereto is a gene involved in adenine auxotrophy of *Starmerella bombicola* and is capable of functioning as an (adenine) auxotrophic selection marker derived from an endogenous gene of *Starmerella bombicola* (endogenous selection marker, self-marker). Accordingly, the mutant strain of the present invention is useful as a host in a method for transforming *Starmerella bombicola,* particularly, as a host in a method for transforming *Starmerella bombicola,* including selecting a transformant by using the presence or absence of adenine auxotrophy as an index. Thus, the present invention also provides a method for transforming *Starmerella bombicola,* including using the mutant strain of the present invention as a host.

In the transformation method of the present invention, a polynucleotide to be introduced into the host is not particularly limited and can be an arbitrary polynucleotide. The polynucleotide may be a polynucleotide derived from the same species of microorganism as that of the host into which it is introduced (i.e., *Starmerella bombicola*), or may be a polynucleotide derived from a different species of microorganism from that of the host. The polynucleotide is preferably a polynucleotide derived from the same species of microorganism as that of the host, or a different species of microorganism from that of the host, provided that genetic exchange has been found to occur between the host and the microorganism in nature, more preferably a polynucleotide derived from the same species of microorganism as that of the host, because the transformant can fall within self-cloning or natural occurrence. The polynucleotide may be isolated from the nature, may be synthesized, or may be produced by use of a genetic engineering approach. The polypeptide can be in the form of single-stranded or double-stranded DNA, RNA, or artificial nucleic acid or can be cDNA, or chemically synthesized DNA containing no intron. The polynucleotide may be codon-optimized for the species of the host. Information on codons which are employed by various organisms are available from Codon Usage Database ([www.kazusa.or.jp/codon/]).

Examples of the approach of introducing the polynucleotide into the host include introduction of a vector or a DNA fragment containing the polynucleotide into the host.

The vector containing the polynucleotide described above may be a vector capable of extrachromosomally proliferating and replicating autonomously or may be a vector which is integrated into chromosomes. Examples of the type of the vector include, but are not particularly limited to, plasmids, cosmids, phages, viruses, YAC, and BAC. Among them, a plasmid vector is preferred. Those skilled in the art can select a suitable vector for the type of the host. The plasmid vector may be prepared depending on the host, or a commercially available product may be used.

Examples of the DNA fragment containing the polynucleotide described above include DNA fragments amplified by PCR and DNA fragments cleaved with restriction enzymes.

In the vector or the DNA fragment, a selection marker such as an antibiotic resistance gene or an auxotrophy-related gene may be further incorporated in order to select a transformant into which the vector or the DNA fragment has been properly introduced. Alternatively, the selection marker may be incorporated into the vector or the DNA fragment as the selection marker and as the polynucleotide to be introduced into the host. Preferably, a gene encoding the phosphoribosyl-glycinamide formyltransferase-like protein of *Starmerella bombicola* or a gene equivalent thereto, more preferably a gene encoding a polypeptide consisting of the amino acid sequence of SEQ ID NO: 2 or a polypeptide consisting of an amino acid sequence having at least 90% identity to the amino acid sequence of SEQ ID NO: 2, further more preferably a gene consisting of the nucleotide sequence of SEQ ID NO: 1 or a gene consisting of a nucleotide sequence having at least 90% identity to the nucleotide sequence of SEQ ID NO: 1, even more preferably a gene consisting of a nucleotide sequence of SEQ ID NO: 1, is used as the selection marker to be incorporated into the vector or the DNA fragment.

When the polynucleotide to be introduced into the host contains a gene, the vector or the DNA fragment preferably further contain the gene as well as a control region operably linked thereto. The control region is a sequence for allowing the gene to be expressed in the host into which the vector or the DNA fragment has been introduced. Examples thereof include expression regulation regions such as promoters and terminators, replication origins, and secretion signal regions for secreting an expressed protein to the outside of cells. The type of the control region is not particularly limited, and a promoter or a secretion signal sequence usually used can be appropriately selected and used depending on the host for introduction. Suitable examples of the control sequence include *Starmerella bombicola-*derived promoters and secretion signal sequences.

The polynucleotide to be introduced into the host, which is contained in the vector or the DNA fragment, may be introduced into the nucleus of the host or may be introduced into the host genome. Examples of the approach of introducing the polynucleotide into the genome include homologous recombination.

Any method usually used such as electroporation, a particle gun method, a lithium acetate method, or a heat treatment method can be used as a method for introducing the vector or the DNA fragment into the host.

A transformant into which the vector or the DNA fragment of interest has been introduced can be selected through the use of the selection marker. For example, when the selection marker is an antibiotic resistance gene, the transformant into which the vector or the DNA fragment of interest has been introduced can be selected by culturing cells in a medium supplemented with the antibiotic. For example, when the selection marker is an auxotrophy-related gene, the transformant into which the vector or the DNA fragment of interest has been introduced can be selected by introducing the vector or the DNA fragment into a host having predetermined auxotrophy, and then using the presence or absence of the auxotrophy as an index. Alternatively, the introduction of the vector or the DNA fragment of interest may be confirmed by examining the DNA sequence of the transformant by PCR or the like.

In a preferred embodiment, in the transformation method of the present invention, a gene encoding the phosphoribosyl-glycinamide formyltransferase-like protein of *Starmerella bombicola* or a gene equivalent thereto is used as a selection marker. The gene is a gene involved in adenine auxotrophy of *Starmerella bombicola.* The mutant strain of the present invention for use as a host in the transformation method of the present invention has adenine auxotrophy, whereas a transformant into which the gene has been introduced becomes adenine non-auxotrophic by canceling the adenine auxotrophy and can thus grow in an adenine-free medium. Accordingly, the transformant into which the vector or the DNA fragment of interest has been introduced can be selected by culturing cells in an adenine-free medium. Since the gene also functions as an endogenous selection marker (self-marker) of *Starmerella bombicola,* the transformant obtained by the transformation method of the present invention with the gene as a selection marker may fall within self-cloning as long as the host is the mutant strain of the present invention containing no foreign gene, preferably a T1 strain, and the polynucleotide to be introduced into the host is derived from the same species of microorganism as that of the host (i.e., *Starmerella bombicola*); and the transformant may fall within natural occurrence as long as the polynucleotide to be introduced into the host is derived from a different species of microorganism from that of the host, provided that genetic exchange has been found to occur between the host and the micoorganism in nature. Thus, in a more preferred embodiment, in the transformation method of the present invention, a gene of the phosphoribosyl-glycinamide formyltransferase-like protein of *Starmerella bombicola* or a gene equivalent thereto is used as the selection marker; the mutant strain of the present invention containing no foreign gene is used as the host; and a polynucleotide derived from the same species of microorganism as that of the host or a polynucleotide derived from a different species of microorganism from that of the host, provided that genetic exchange has been found to occur between the host and the microorganism in nature, further more preferably a polynucleotide derived from the same species of microorganism as that of the host, is used as the polynucleotide to be introduced into the host.

The gene encoding the phosphoribosyl-glycinamide formyltransferase-like protein of *Starmerella bombicola* or the gene equivalent thereto as an endogenous selection marker (self-marker) is preferably an isolated gene. In this context, the isolated gene means a gene present independently from genomic DNA of an organism from which the gene is derived, and include a gene separated from genomic DNA of an organism from which the gene is derived or a gene synthesized in a genetic engineering or synthetic manner. The gene encoding the phosphoribosyl-glycinamide formyltransferase-like protein or the gene equivalent thereto can be prepared by extracting genomic DNA from *Starmerella bombicola* having the gene by a routine method, or extracting RNA therefrom and synthesizing cDNA by reverse transcription. The gene encoding the phosphoribosyl-glycinamide formyltransferase-like protein of *Starmerella bombicola* can be prepared from, for example, a *Starmerella bombicola* NBRC10243 strain. The gene equivalent to the gene encoding the phosphoribosyl-glycinamide formyltransferase-like protein of *Starmerella bombicola* can be prepared by modifying the gene encoding the phosphoribosyl-glycinamide formyltransferase-like protein of *Starmerella bombicola* by use of various mutagenesis techniques known in the art. Alternatively, the gene encoding the phosphoribosyl-glycinamide formyltransferase-like protein of *Starmerella bombicola* or the gene equivalent thereto can be prepared by synthesizing a corresponding nucleotide sequence in a genetic engineering or synthetic manner based on the amino acid sequence of SEQ ID NO: 2 or an amino acid sequence having at least 90% identity thereto.

The transformation method of the present invention can provide a transformant having a polynucleotide of interest that is introduced into the mutant strain of the present invention by, for example, introducing, into the mutant strain of the present invention as a host, a polynucleotide of interest to be introduced into the host and the gene encoding the phosphoribosyl-glycinamide formyltransferase-like protein of *Starmerella bombicola* or the gene equivalent thereto as a selection marker, and selecting adenine non-auxotrophic cells.

Alternatively, the transformation method of the present invention can provide a transformant having a deleted or inactivated endogenous polynucleotide of interest in the genome of the mutant strain of the present invention by, for example, introducing, into the genome of the mutant strain of the present invention as a host, the gene encoding the phosphoribosyl-glycinamide formyltransferase-like protein of *Starmerella bombicola* or the gene equivalent thereto as a selection marker so as to delete or inactivate the endogenous polynucleotide of interest in the host genome, and selecting adenine non-auxotrophic cells. In this context, the endogenous polynucleotide of interest is not particularly limited as long as it is a polynucleotide contained in the host genome before transformation, and may be any polynucleotide.

### (4. Method for producing glycolipid)

*Starmerella bombicola* is known to produce glycolipids with hydrocarbon chains, fatty acids, and the like having various chain lengths as substrates. Accordingly, the mutant strain of the present invention of the section 2 or the transformant obtained by the transformation method of the present invention of the section 3 (hereinafter, referred to as the transformant of the present invention) can be cultured together with a substrate having a proper chain length to efficiently produce a glycolipid containing a constituent hydrocarbon chain, fatty acid, or the like having the desired chain length. Thus, the present invention also provides a method for producing a glycolipid, including culturing the mutant strain of the present invention or the transformant of the present invention.

The glycolipid is not particularly limited as long as the glycolipid is capable of being produced by the mutant strain of the present invention or the transformant of the present invention. Examples thereof preferably include glycolipids containing glucose or an acetylated form thereof as a sugar or a sugar constituting a sugar chain, more preferably include glycolipids containing glucose, sophorose, cellobiose, or an acetylated form thereof as a sugar or a sugar chain, further more preferably include sophorolipid, Bola sophorolipid, Bola sophoroside, alkyl sophoroside, alkyl glucoside, Bola glucoside, acidic glucolipid, and cellobiose lipid, even more preferably include sophorolipid, Bola sophoroside, and alkyl sophoroside, and even more preferably sophorolipid and Bola sophoroside.

The transformant for use in the method for producing a glycolipid according to the present invention is not particularly limited as long as the transformant is obtained by the transformation method of the present invention. For example, a transformant modified so as to improve the ability of *Starmerella bombicola* to produce glycolipids is preferably used. Examples of the transformant modified so as to improve the ability of *Starmerella bombicola* to produce glycolipids include transformants in which expression of a polypeptide involved in suppression of the ability of *Starmerella bombicola* to produce the glycolipid of interest is suppressed or the polypeptide is inactivated, transformants in which expression of a polypeptide involved in improvement of the ability of *Starmerella bombicola* to produce the glycolipid of interest is enhanced, and transformants in which expression of various enzymes for production of the glycolipid of interest in *Starmerella bombicola* is enhanced.

In a preferred embodiment, the transformant for use in the method for producing a glycolipid according to the present invention is a transformant in which expression of a polypeptide consisting of the amino acid sequence of SEQ ID NO: 23 or an amino acid sequence having at least 90% identity to the amino acid sequence of SEQ ID NO: 23 is suppressed or the polypeptide is inactivated by the transformation method of the present invention. The polypeptide consisting of the amino acid sequence of SEQ ID NO: 23 or an amino acid sequence having at least 90% identity to the amino acid sequence of SEQ ID NO: 23 is a putative transcription factor protein, more preferably a putative transcription factor protein having two zinc finger C2H2-type DNA binding domains. The presence or absence of the zinc finger C2H2-type DNA binding domain in a certain polypeptide can be confirmed, for example, by analyzing an amino acid sequence using CD search of NCBI, though the method for analyzing the presence or absence of the domain is not limited thereto. The transformant is preferably a transformant in which a gene encoding the polypeptide consisting of the amino acid sequence of SEQ ID NO: 23 or an amino acid sequence having at least 90% identity to the amino acid sequence of SEQ ID NO: 23 is deleted or inactivated, more preferably a transformant in which a gene consisting of the nucleotide sequence of SEQ ID NO: 22 or a nucleotide sequence having at least 90% identity to the nucleotide sequence of SEQ ID NO: 22 is deleted or inactivated, further more preferably a transformant in which a gene consisting of the nucleotide sequence of SEQ ID NO: 22 or a gene which consists of a nucleotide sequence having at least 90% identity to the nucleotide sequence of SEQ ID NO: 22 and encodes the polypeptide consisting of the amino acid sequence of SEQ ID NO: 23 or an amino acid sequence having at least 90% identity to the amino acid sequence of SEQ ID NO: 23 is deleted or inactivated. In this context, the polypeptide consisting of the amino acid sequence of SEQ ID NO: 23 is encoded by the nucleotide sequence of SEQ ID NO: 22 in *Starmerella bombicola,* and a *Starmerella bombicola* mutant strain in which expression of the polypeptide is suppressed or the polypeptide is inactivated has been reported to have the improved ability to produce sophorolipid as compared with a parent strain (JP-B-6725506). Specific examples of the transformant include a *Starmerella bombicola* T1Δseq1::ade strain shown in Examples described later. The T1Δseq1::ade strain is considered to correspond to a strain that fall within self-cloning because a gene consisting of the nucleotide sequence of SEQ ID NO: 1 in the genome of a *Starmerella bombicola* NBRC10243 strain is inserted so as to replace a gene consisting of the nucleotide sequence of SEQ ID NO: 22 in the genome of a T1 strain by transformation with the T1 strain, which is considered to correspond to a spontaneous mutant strain, as a host.

In another preferred embodiment, the transformant for use in the method for producing a glycolipid according to the present invention is a transformant in which expression of FAO1 (fatty alcohol oxidase 1) or a protein equivalent thereto is suppressed or the FAO1 or a protein equivalent thereto is inactivated by the transformation method of the present invention. In this context, the "FAO1" is FAO1 of *Starmerella bombicola* and is a polypeptide having oxidase activity for aliphatic alcohols, preferably a polypeptide consisting of the amino acid sequence of SEQ ID NO: 35. The "protein equivalent to the FAO1" is a protein having the same function as that of the FAO1 of *Starmerella bombicola* and includes a homolog or an ortholog of the FAO1 of *Starmerella bombicola,* or a mutant thereof. The protein equivalent to the FAO1 is preferably a polypeptide consisting of an amino acid sequence having at least 90% identity to the amino acid sequence of SEQ ID NO: 35. Examples of the amino acid sequence having at least 90% identity to the amino acid sequence of SEQ ID NO: 35 include an amino acid sequence obtained by deletion, substitution, addition, or insertion of one or several amino acids in the amino acid sequence of SEQ ID NO: 35. The transformant is preferably a transformant in which a gene encoding the polypeptide consisting of the amino acid sequence of SEQ ID NO: 35 or an amino acid sequence having at least 90% identity to the amino acid sequence of SEQ ID NO: 35 is deleted or inactivated, more preferably a transformant in which a gene consisting of the nucleotide sequence of SEQ ID NO: 34 or a gene consisting of a nucleotide sequence having at least 90% identity to the nucleotide sequence of SEQ ID NO: 34 is deleted or inactivated, further more preferably a transformant in which a gene consisting of the nucleotide sequence of SEQ ID NO: 34 or a gene which consists of a nucleotide sequence having at least 90% identity to the nucleotide sequence of SEQ ID NO: 34 and encodes the polypeptide consisting of the amino acid sequence of SEQ ID NO: 35 or an amino acid sequence having at least 90% identity to the amino acid sequence of SEQ ID NO: 35 is deleted or inactivated. The polypeptide consisting of the amino acid sequence of SEQ ID NO: 35 is encoded by the nucleotide sequence of SEQ ID NO: 34 in *Starmerella bombicola,* and a *Starmerella bombicola* mutant strain in which expression of the polypeptide is suppressed or the polypeptide is inactivated has been reported to become capable of producing Bola sophoroside, alkyl sophoroside, alkyl glucoside, and Bola glucoside (Appl Microbiol Biotechnol. 2016 Nov; 100 (22): 9519-9528). Specific examples of the transformant include a *Starmerella bombicola* T1Δfao1::ade strain shown in Examples described later. The T1Δfao1::ade strain is considered to correspond to a strain that fall within self-cloning because a gene consisting of the nucleotide sequence of SEQ ID NO: 1 in the genome of a *Starmerella bombicola* NBRC10243 strain is inserted so as to replace fao1 gene consisting of the nucleotide sequence of SEQ ID NO: 34 in the genome of a T1 strain by transformation with the T1 strain, which is considered to correspond to a spontaneous mutant strain, as a host.

In another preferred embodiment, the transformant for use in the method for producing a glycolipid according to the present invention is a transformant in which expression of CYP52M1 (CYP52M1 cytochrome P450 monooxygenase) or a protein equivalent thereto is suppressed or the CYP52M1 or a protein equivalent thereto is inactivated by the transformation method of the present invention. In this context, the "CYP52M1" is CYP52M1 of *Starmerella bombicola* and is a polypeptide having hydroxylation activity at ω or ω-1 position of fatty acid or aliphatic alcohol, preferably a polypeptide consisting of the amino acid sequence of SEQ ID NO: 37. The "protein equivalent to the CYP52M1" is a protein having the same function as that of the CYP52M1 of *Starmerella bombicola* and includes a homolog or an ortholog of the CYP52M1 of *Starmerella bombicola,* or a mutant thereof. The protein equivalent to the CYP52M1 is preferably a polypeptide consisting of an amino acid sequence having at least 90% identity to the amino acid sequence of SEQ ID NO: 37. Examples of the amino acid sequence having at least 90% identity to the amino acid sequence of SEQ ID NO: 37 include an amino acid sequence obtained by deletion, substitution, addition, or insertion of one or several amino acids in the amino acid sequence of SEQ ID NO: 37. The transformant is preferably a transformant in which a gene encoding the polypeptide consisting of the amino acid sequence of SEQ ID NO: 37 or an amino acid sequence having at least 90% identity to the amino acid sequence of SEQ ID NO: 37 is deleted or inactivated, more preferably a transformant in which a gene consisting of the nucleotide sequence of SEQ ID NO: 36 or a gene consisting of a nucleotide sequence having at least 90% identity to the nucleotide sequence of SEQ ID NO: 36 is deleted or inactivated, further more preferably a transformant in which a gene consisting of the nucleotide sequence of SEQ ID NO: 36 or a gene which consists of a nucleotide sequence having at least 90% identity to the nucleotide sequence of SEQ ID NO: 36 and encodes the polypeptide consisting of the amino acid sequence of SEQ ID NO: 37 or an amino acid sequence having at least 90% identity to the amino acid sequence of SEQ ID NO: 37 is deleted or inactivated. The polypeptide consisting of the amino acid sequence of SEQ ID NO: 37 is encoded by the nucleotide sequence of SEQ ID NO: 36 in *Starmerella bombicola. A Starmerella bombicola* mutant strain in which cyp52M1 gene and fao1 gene are disrupted has been reported to have good productivity of Bola sophoroside, and suppress by-production of sophorolipid (WO 2020/104582).

In another preferred embodiment, the transformant for use in the method for producing a glycolipid according to the present invention is a transformant in which expression of CYP52N1 (CYP52N1 cytochrome P450 monooxygenase) or a protein equivalent thereto is suppressed or the CYP52N1 or a protein equivalent thereto is inactivated by the transformation method of the present invention. In this context, the "CYP52N1" is CYP52N1 of *Starmerella bombicola* and is, as in the CYP52M1, a polypeptide having hydroxylation activity at ω or ω-1 position of fatty acid or aliphatic alcohol (Inge N. A. Van Bogaert et al., FEMS Yeast Res., 2009, 9(1): 87-94), preferably a polypeptide consisting of the amino acid sequence of SEQ ID NO: 39. The "protein equivalent to the CYP52N1" is a protein having the same function as that of the CYP52N1 of *Starmerella bombicola* and includes a homolog or an ortholog of the CYP52N1 of *Starmerella bombicola,* or a mutant thereof. The protein equivalent to the CYP52N1 is preferably a polypeptide consisting of an amino acid sequence having at least 90% identity to the amino acid sequence of SEQ ID NO: 39. Examples of the amino acid sequence having at least 90% identity to the amino acid sequence of SEQ ID NO: 39 include an amino acid sequence obtained by deletion, substitution, addition, or insertion of one or several amino acids in the amino acid sequence of SEQ ID NO: 39. The transformant is preferably a transformant in which a gene encoding the polypeptide consisting of the amino acid sequence of SEQ ID NO: 39 or an amino acid sequence having at least 90% identity to the amino acid sequence of SEQ ID NO: 39 is deleted or inactivated, more preferably a transformant in which a gene consisting of the nucleotide sequence of SEQ ID NO: 38 or a gene consisting of a nucleotide sequence having at least 90% identity to the nucleotide sequence of SEQ ID NO: 38 is deleted or inactivated, further more preferably a transformant in which a gene consisting of the nucleotide sequence of SEQ ID NO: 38 or a gene which consists of a nucleotide sequence having at least 90% identity to the nucleotide sequence of SEQ ID NO: 38 and encodes the polypeptide consisting of the amino acid sequence of SEQ ID NO: 39 or an amino acid sequence having at least 90% identity to the amino acid sequence of SEQ ID NO: 39 is deleted or inactivated. The polypeptide consisting of the amino acid sequence of SEQ ID NO: 39 is encoded by the nucleotide sequence of SEQ ID NO: 38 in *Starmerella bombicola.*

In another preferred embodiment, the transformant for use in the method for producing a glycolipid according to the present invention is a transformant in which expression of CYP52E3 (CYP52E3 cytochrome P450 monooxygenase) or a protein equivalent thereto is suppressed or the CYP52E3 or a protein equivalent thereto is inactivated by the transformation method of the present invention. In this context, the "CYP52E3" is CYP52E3 of *Starmerella bombicola* and is, as in the CYP52M1, a polypeptide having hydroxylation activity at ω or ω-1 position of fatty acid or aliphatic alcohol (Inge N. A. Van Bogaert et al., FEMS Yeast Res., 2009, 9(1): 87-94), preferably a polypeptide consisting of the amino acid sequence of SEQ ID NO: 41. The "protein equivalent to the CYP52E3" is a protein having the same function as that of the CYP52E3 of *Starmerella bombicola* and includes a homolog or an ortholog of the CYP52E3 of *Starmerella bombicola,* or a mutant thereof. The protein equivalent to the CYP52E3 is preferably a polypeptide consisting of an amino acid sequence having at least 90% identity to the amino acid sequence of SEQ ID NO: 41. Examples of the amino acid sequence having at least 90% identity to the amino acid sequence of SEQ ID NO: 41 include an amino acid sequence obtained by deletion, substitution, addition, or insertion of one or several amino acids in the amino acid sequence of SEQ ID NO: 41. The transformant is preferably a transformant in which a gene encoding the polypeptide consisting of the amino acid sequence of SEQ ID NO: 41 or an amino acid sequence having at least 90% identity to the amino acid sequence of SEQ ID NO: 41 is deleted or inactivated, more preferably a transformant in which a gene consisting of the nucleotide sequence of SEQ ID NO: 40 or a gene consisting of a nucleotide sequence having at least 90% identity to the nucleotide sequence of SEQ ID NO: 40 is deleted or inactivated, further more preferably a transformant in which a gene consisting of the nucleotide sequence of SEQ ID NO: 40 or a gene which consists of a nucleotide sequence having at least 90% identity to the nucleotide sequence of SEQ ID NO: 40 and encodes the polypeptide consisting of the amino acid sequence of SEQ ID NO: 41 or an amino acid sequence having at least 90% identity to the amino acid sequence of SEQ ID NO: 41 is deleted or inactivated. The polypeptide consisting of the amino acid sequence of SEQ ID NO: 41 is encoded by the nucleotide sequence of SEQ ID NO: 40 in *Starmerella bombicola.*

In another preferred embodiment, the transformant for use in the method for producing a glycolipid according to the present invention is a transformant in which expression of UGTB1 (UDP-glucosyltransferase B1) or a protein equivalent thereto is suppressed or the UGTB1 or a protein equivalent thereto is inactivated by the transformation method of the present invention. In this context, the "UGTB1" is UGTB1 of *Starmerella bombicola* and is a polypeptide having transglycosylation activity to hydroxylated fatty acids from UDP-glucose as a donor, preferably a polypeptide consisting of the amino acid sequence of SEQ ID NO: 43. The "protein equivalent to the UGTB1" is a protein having the same function as that of the UGTB1 of *Starmerella bombicola* and includes a homolog or an ortholog of the UGTB1 of *Starmerella bombicola,* or a mutant thereof. The protein equivalent to the UGTB1 is preferably a polypeptide consisting of an amino acid sequence having at least 90% identity to the amino acid sequence of SEQ ID NO: 43. Examples of the amino acid sequence having at least 90% identity to the amino acid sequence of SEQ ID NO: 43 include an amino acid sequence obtained by deletion, substitution, addition, or insertion of one or several amino acids in the amino acid sequence of SEQ ID NO: 43. The transformant is preferably a transformant in which a gene encoding the polypeptide consisting of the amino acid sequence of SEQ ID NO: 43 or an amino acid sequence having at least 90% identity to the amino acid sequence of SEQ ID NO: 43 is deleted or inactivated, more preferably a transformant in which a gene consisting of the nucleotide sequence of SEQ ID NO: 42 or a gene consisting of a nucleotide sequence having at least 90% identity to the nucleotide sequence of SEQ ID NO: 42 is deleted or inactivated, further more preferably a transformant in which a gene consisting of the nucleotide sequence of SEQ ID NO: 42 or a gene which consists of a nucleotide sequence having at least 90% identity to the nucleotide sequence of SEQ ID NO: 42 and encodes the polypeptide consisting of the amino acid sequence of SEQ ID NO: 43 or an amino acid sequence having at least 90% identity to the amino acid sequence of SEQ ID NO: 43 is deleted or inactivated. The polypeptide consisting of the amino acid sequence of SEQ ID NO: 43 is encoded by the nucleotide sequence of SEQ ID NO: 42 in *Starmerella bombicola.* A *Starmerella bombicola* mutant strain in which ugtB1 gene is disrupted has been reported to become capable of producing glucolipid (Sofie Lodens et al., Biotechnol Bioeng., 2020, 117(2): 453-465). Further, a *Starmerella bombicola* mutant strain in which ugtB1 gene, fao1 gene, and cyp52M1 gene are disrupted has been reported to become capable of producing alkyl glucoside and alkyl sophoroside (WO 2021/229017).

In another preferred embodiment, the transformant for use in the method for producing a glycolipid according to the present invention is a transformant in which expression of UGTA1 (UDP-glucosyltransferase A1) or a protein equivalent thereto is suppressed or the UGTA1 or a protein equivalent thereto is inactivated by the transformation method of the present invention. In this context, the "UGTA1" is UGTA1 of *Starmerella bombicola* and is, as in the UGTB1, a polypeptide having transglycosylation activity to hydroxylated fatty acids from UDP-glucose as a donor (Karen M. J. Saerens et al., FEMS Yeast Res., 2015, 15(7): fov075), preferably a polypeptide consisting of the amino acid sequence of SEQ ID NO: 45. The "protein equivalent to the UGTA1" is a protein having the same function as that of the UGTA1 of *Starmerella bombicola* and includes a homolog or an ortholog of the UGTA1 of *Starmerella bombicola,* or a mutant thereof. The protein equivalent to the UGTA1 is preferably a polypeptide consisting of an amino acid sequence having at least 90% identity to the amino acid sequence of SEQ ID NO: 45. Examples of the amino acid sequence having at least 90% identity to the amino acid sequence of SEQ ID NO: 45 include an amino acid sequence obtained by deletion, substitution, addition, or insertion of one or several amino acids in the amino acid sequence of SEQ ID NO: 45. The transformant is preferably a transformant in which a gene encoding the polypeptide consisting of the amino acid sequence of SEQ ID NO: 45 or an amino acid sequence having at least 90% identity to the amino acid sequence of SEQ ID NO: 45 is deleted or inactivated, more preferably a transformant in which a gene consisting of the nucleotide sequence of SEQ ID NO: 44 or a gene consisting of a nucleotide sequence having at least 90% identity to the nucleotide sequence of SEQ ID NO: 44 is deleted or inactivated, further more preferably a transformant in which a gene consisting of the nucleotide sequence of SEQ ID NO: 44 or a gene which consists of a nucleotide sequence having at least 90% identity to the nucleotide sequence of SEQ ID NO: 44 and encodes the polypeptide consisting of the amino acid sequence of SEQ ID NO: 45 or an amino acid sequence having at least 90% identity to the amino acid sequence of SEQ ID NO: 45 is deleted or inactivated. The polypeptide consisting of the amino acid sequence of SEQ ID NO: 45 is encoded by the nucleotide sequence of SEQ ID NO: 44 in *Starmerella bombicola.*

In another preferred embodiment, the transformant for use in the method for producing a glycolipid according to the present invention is a transformant in which expression of AT (acetyl transferase) or a protein equivalent thereto is suppressed or the AT or a protein equivalent thereto is inactivated by the transformation method of the present invention. In this context, the "AT" is AT of *Starmerella bombicola* and is a polypeptide having acetylation activity of OH moiety of sugar, preferably a polypeptide consisting of the amino acid sequence of SEQ ID NO: 47. The "protein equivalent to the AT" is a protein having the same function as that of the AT of *Starmerella bombicola* and includes a homolog or an ortholog of the AT of *Starmerella bombicola,* or a mutant thereof. The protein equivalent to the AT is preferably a polypeptide consisting of an amino acid sequence having at least 90% identity to the amino acid sequence of SEQ ID NO: 47. Examples of the amino acid sequence having at least 90% identity to the amino acid sequence of SEQ ID NO: 47 include an amino acid sequence obtained by deletion, substitution, addition, or insertion of one or several amino acids in the amino acid sequence of SEQ ID NO: 47. The transformant is preferably a transformant in which a gene encoding the polypeptide consisting of the amino acid sequence of SEQ ID NO: 47 or an amino acid sequence having at least 90% identity to the amino acid sequence of SEQ ID NO: 47 is deleted or inactivated, more preferably a transformant in which a gene consisting of the nucleotide sequence of SEQ ID NO: 46 or a gene consisting of a nucleotide sequence having at least 90% identity to the nucleotide sequence of SEQ ID NO: 46 is deleted or inactivated, further more preferably a transformant in which a gene consisting of the nucleotide sequence of SEQ ID NO: 46 or a gene which consists of a nucleotide sequence having at least 90% identity to the nucleotide sequence of SEQ ID NO: 46 and encodes the polypeptide consisting of the amino acid sequence of SEQ ID NO: 47 or an amino acid sequence having at least 90% identity to the amino acid sequence of SEQ ID NO: 47 is deleted or inactivated. The polypeptide consisting of the amino acid sequence of SEQ ID NO: 47 is encoded by the nucleotide sequence of SEQ ID NO: 46 in *Starmerella bombicola.* A *Starmerella bombicola* mutant strain in which at gene is disrupted has been reported to produce non-acetylated sophorolipid (Karen M. J. Saerens et al., Biotechnol Bioeng., 2011, 108(12): 2923-2931).

In another preferred embodiment, the transformant for use in the method for producing a glycolipid according to the present invention is a transformant in which expression of SBLE (*Starmerella Bombicola* Lactone Esterase) or a protein equivalent thereto is suppressed or the SBLE or a protein equivalent thereto is inactivated by the transformation method of the present invention. In this context, the "SBLE" is SBLE of *Starmerella bombicola* and is a polypeptide having lactonization activity of sophorolipid, preferably a polypeptide consisting of the amino acid sequence of SEQ ID NO: 49. The "protein equivalent to the SBLE" is a protein having the same function as that of the SBLE of *Starmerella bombicola* and includes a homolog or an ortholog of the SBLE of *Starmerella bombicola,* or a mutant thereof. The protein equivalent to the SBLE is preferably a polypeptide consisting of an amino acid sequence having at least 90% identity to the amino acid sequence of SEQ ID NO: 49. Examples of the amino acid sequence having at least 90% identity to the amino acid sequence of SEQ ID NO: 49 include an amino acid sequence obtained by deletion, substitution, addition, or insertion of one or several amino acids in the amino acid sequence of SEQ ID NO: 49. The transformant is preferably a transformant in which a gene encoding the polypeptide consisting of the amino acid sequence of SEQ ID NO: 49 or an amino acid sequence having at least 90% identity to the amino acid sequence of SEQ ID NO: 49 is deleted or inactivated, more preferably a transformant in which a gene consisting of the nucleotide sequence of SEQ ID NO: 48 or a gene consisting of a nucleotide sequence having at least 90% identity to the nucleotide sequence of SEQ ID NO: 48 is deleted or inactivated, further more preferably a transformant in which a gene consisting of the nucleotide sequence of SEQ ID NO: 48 or a gene which consists of a nucleotide sequence having at least 90% identity to the nucleotide sequence of SEQ ID NO: 48 and encodes the polypeptide consisting of the amino acid sequence of SEQ ID NO: 49 or an amino acid sequence having at least 90% identity to the amino acid sequence of SEQ ID NO: 49 is deleted or inactivated. The polypeptide consisting of the amino acid sequence of SEQ ID NO: 49 is encoded by the nucleotide sequence of SEQ ID NO: 48 in *Starmerella bombicola.* A *Starmerella bombicola* mutant strain in which sble gene is disrupted has been reported to improve the percentage of acidic sophorolipid (Katarzyna Ciesielska et al., Appl Microbiol Biotechnol., 2016, 100(22): 9529-954). Further, a *Starmerella bombicola* mutant strain in which sble gene and at gene are disrupted has been reported to become capable of producing non-acetylated Bola sophorolipid (WO 2015/028278).

In another preferred embodiment, the transformant for use in the method for producing a glycolipid according to the present invention is a transformant in which expression of MDR1 (multidrug resistance protein 1) or a protein equivalent thereto is enhanced by the transformation method of the present invention. In this context, the "MDR1" is MDR1 of *Starmerella bombicola* and is a transporter polypeptide responsible for intracellular and extracellular transport of sophorolipid, preferably a polypeptide consisting of the amino acid sequence of SEQ ID NO: 51. The "protein equivalent to the MDR1" is a protein having the same function as that of the MDR1 of *Starmerella bombicola* and includes a homolog or an ortholog of the MDR1 of *Starmerella bombicola,* or a mutant thereof. The protein equivalent to the MDR1 is preferably a polypeptide consisting of an amino acid sequence having at least 90% identity to the amino acid sequence of SEQ ID NO: 51. Examples of the amino acid sequence having at least 90% identity to the amino acid sequence of SEQ ID NO: 51 include an amino acid sequence obtained by deletion, substitution, addition, or insertion of one or several amino acids in the amino acid sequence of SEQ ID NO: 51. The transformant is preferably a transformant in which expression of a gene encoding the polypeptide consisting of the amino acid sequence of SEQ ID NO: 51 or an amino acid sequence having at least 90% identity to the amino acid sequence of SEQ ID NO: 51 is enhanced, more preferably a transformant in which expression of a gene consisting of the nucleotide sequence of SEQ ID NO: 50 or a gene consisting of a nucleotide sequence having at least 90% identity to the nucleotide sequence of SEQ ID NO: 50 is enhanced, further more preferably a transformant in which expression of a gene consisting of the nucleotide sequence of SEQ ID NO: 50 or a gene which consists of a nucleotide sequence having at least 90% identity to the nucleotide sequence of SEQ ID NO: 50 and encodes the polypeptide consisting of the amino acid sequence of SEQ ID NO: 51 or an amino acid sequence having at least 90% identity to the amino acid sequence of SEQ ID NO: 51 is enhanced. The polypeptide consisting of the amino acid sequence of SEQ ID NO: 51 is encoded by the nucleotide sequence of SEQ ID NO: 50 in *Starmerella bombicola.* A *Starmerella bombicola* mutant strain in which mdr1 gene is disrupted has been reported to significantly reduce productivity of sophorolipid and Bola sophorolipid (Silke Claus et al., BMC Genomics, 2022, 23(1): 22).

In another preferred embodiment, the transformant for use in the method for producing a glycolipid according to the present invention is a transformant in which expression of MFE-2 (multifunctional enzyme type 2) or a protein equivalent thereto is suppressed or the MFE-2 or a protein equivalent thereto is inactivated by the transformation method of the present invention. In this context, the "MFE-2" is MFE-2 of *Starmerella bombicola* and is a polypeptide involved in beta oxidative decomposition of fatty acid. The "protein equivalent to the MFE-2" is a protein having the same function as that of the MFE-2 of *Starmerella bombicola* and includes a homolog or an ortholog of the MFE-2 of *Starmerella bombicola,* or a mutant thereof. A *Starmerella bombicola* mutant strain in which mfe-2 gene is disrupted has been reported to produce sophorolipid having a medium-chain fatty acid backbone (Inge N. A. Van Bogaert et al., FEMS Yeast Res., 2009, 9(4): 610-617).

In the method for producing a glycolipid according to the present invention, the mutant strain of the present invention or the transformant of the present invention is cultured in a medium containing a substrate such as fatty acid, fatty acid alkyl ester, triacylglycerol, diacylglycerol, monoacylglycerol, fat or oil, alkane, alkene, alkyne, and alcohol. The glycolipid can be recovered from the medium after culture and appropriately purified, if necessary, to produce a glycolipid.

A common medium containing a carbon source, a nitrogen source, an inorganic salt, and optionally an organic micronutrient such as an amino acid and a vitamin cam be used as the medium for use in the culture. The medium may be any of a synthetic medium and a natural medium.

The carbon source and the nitrogen source contained in the medium may be of any type utilizable by the mutant strain or the transformant to be cultured. Examples of the carbon source include: sugars such as glucose, glycerol, fructose, sucrose, maltose, mannose, galactose, starch hydrolysates, and molasses; organic acids such as acetic acid and citric acid; and alcohols such as ethanol. Any one of these carbon sources can be used alone, or two or more thereof can be used in combination. Examples of the nitrogen source include: ammonia; ammonium salts such as ammonium sulfate, ammonium carbonate, ammonium chloride, ammonium phosphate, and ammonium acetate; nitrate; and urea.

Examples of the inorganic salt include phosphate, magnesium salt, calcium salt, iron salt, and manganese salt. Examples of the organic micronutrient include amino acids, vitamins, fatty acids, nucleic acids, and peptone, casamino acid, yeast extracts, and soybean protein degradants containing these. In the case of using an auxotrophic mutant strain or transformant which requires an amino acid or the like for growth, the required nutrient may be added to the medium.

Preferred examples of the substrate which may be contained in the medium include C12 to 22 fatty acid and alkyl ester thereof, triacylglycerol, diacylglycerol, and monoacylglycerol containing C12 to 22 fatty acid or alkyl ester thereof, fat or oil containing C12 to 22 fatty acid or alkyl ester thereof, C12 to 22 alkane, C12 to 22 alkene, C12 to 22 alkyne, and C12 to 22 alcohol. More preferred examples thereof include C12 to 18 fatty acid and alkyl ester thereof, triacylglycerol, diacylglycerol, and monoacylglycerol containing C12 to C18 fatty acid or alkyl ester thereof, fat or oil containing C12 to C18 fatty acid or alkyl ester thereof, C12 to 18 alkane, C12 to 18 alkene, C12 to 18 alkyne, and C12 to 18 alcohol. Further more preferred examples thereof include C12 to C18 fatty acid and alkyl ester thereof, triacylglycerol containing C12 to C18 fatty acid or alkyl ester thereof, and C12 to C18 alcohol.

More detailed examples of the substrate include, but are not limited to: the C12 to 22 fatty acid, which may be saturated fatty acid or unsaturated fatty acid and may be linear fatty acid or branched fatty acid, including lauric acid, tridecylic acid, myristic acid, pentadecylic acid, palmitic acid, margaric acid, stearic acid, isostearic acid, nonadecylic acid, arachidic acid, behenic acid, palmitoleic acid, oleic acid, linolic acid, α-linolenic acid, γ-linolenic acid, arachidonic acid, eicosapentaenoic acid, and docosahexaenoic acid; the alkyl ester of the C12 to 22 fatty acid, including alkyl ester having 1 to 4 carbon atoms, preferably methyl ester and ethyl ester, of the fatty acid; and the fat or oil containing C12 to 22 fatty acid or alkyl ester thereof, including coconut oil, palm oil, palm kernel oil, olive oil, rapeseed oil, rice bran oil, soybean oil, castor oil, and madhuca oil.

Examples of the C12 to 22 alkane, which may be linear or branched, include dodecane, tridecane, tetradecane, pentadecane, hexadecane, heptadecane, octadecane, nonadecane, icosane, heneicosane, and docosane. Examples of the C12 to 22 alkene, which may be linear or branched, include 1-dodecene, 1-tridecene, 1-tetradecene, 1-pentadecene, 1-hexadecene, 1-heptadecene, 1-octadecene, 1-nonadecene, 1-icosene, 1-heneicosene, and 1-docosene. Examples of the C12 to 22 alkyne, which may be linear or branched, include 1-dodecyne, 1-tridecyne, 1-tetradecyne, 1-pentadecyne, 1-hexadecyne, 1-heptadecyne, 1-octadecyne, 1-nonadecyne, 1-icosyne, 1-heneicosyne, and 1-docosyne.

Examples of the C12 to 22 alcohol, which may be saturated alcohol or unsaturated alcohol and may be linear alcohol or branched alcohol, include lauryl alcohol, tridecyl alcohol, myristyl alcohol, pentadecyl alcohol, cetyl alcohol, heptadecyl alcohol, stearyl alcohol, isostearyl alcohol, nonadecyl alcohol, arachidyl alcohol, behenyl alcohol, palmitoleyl alcohol, oleyl alcohol, linoleyl alcohol, and linolenyl alcohol.

Any one of the substrates listed above can be used alone, or two or more thereof can be used in combination. Preferably, fatty acid having any chain length of from C12 to C18 or alkyl ester thereof, or triacylglycerol, diacylglycerol, monoacylglycerol, or fat or oil containing it, or alkane, alkene, alkyne, or alcohol having any chain length of from C12 to 18 is used. More preferably, fatty acid having any chain length of from C12 to C18 or alkyl ester thereof, or alcohol having any chain length of from C12 to 18 is used. Further more preferably, fatty acid having any chain length of from C16 to C18 or alkyl ester thereof, or alcohol having any chain length of from C16 to 18 is used. Even more preferably, C18 fatty acid or alkyl ester thereof, or C18 alcohol is used. Even more preferably, oleic acid or alkyl ester thereof, or oleyl alcohol is used. Rapeseed oil is also preferably used which is composed mainly of triacylglycerol rich in oleic acid as constituent fatty acid.

The content of the substrate (at the time of addition of the substrate) which may be contained in the medium is preferably 0.1 mass/volume% or more, more preferably 0.5 mass/volume% or more, further more preferably 1 mass/volume% or more, even more preferably 2 mass/volume% or more, even more preferably 3 mass/volume% or more, even more preferably 5 mass/volume% or more, from a viewpoint of amount of glycolipid produced, and preferably 40 mass/volume% or less, more preferably 30 mass/volume% or less, further more preferably 25 mass/volume% or less, even more preferably 20 mass/volume% or less, even more preferably 15 mass/volume% or less, even more preferably 10 mass/volume% or less, from a viewpoint of glycolipid production efficiency. Alternatively, the content is preferably from 0.1 to 40 mass/volume%, from 0.1 to 30 mass/volume%, from 0.1 to 25 mass/volume%, from 0.1 to 20 mass/volume%, from 0.1 to 15 mass/volume%, from 0.1 to 10 mass/volume%, from 0.5 to 40 mass/volume%, from 0.5 to 30 mass/volume%, from 0.5 to 25 mass/volume%, from 0.5 to 20 mass/volume%, from 0.5 to 15 mass/volume%, from 0.5 to 10 mass/volume%, from 1 to 40 mass/volume%, from 1 to 30 mass/volume%, from 1 to 25 mass/volume%, from 1 to 20 mass/volume%, from 1 to 15 mass/volume%, from 1 to 10 mass/volume%, from 2 to 40 mass/volume%, from 2 to 30 mass/volume%, from 2 to 25 mass/volume%, from 2 to 20 mass/volume%, from 2 to 15 mass/volume%, from 2 to 10 mass/volume%, from 3 to 40 mass/volume%, from 3 to 30 mass/volume%, from 3 to 25 mass/volume%, from 3 to 20 mass/volume%, from 3 to 15 mass/volume%, from 3 to 10 mass/volume%, from 5 to 40 mass/volume%, from 5 to 30 mass/volume%, from 5 to 25 mass/volume%, from 5 to 20 mass/volume%, from 5 to 15 mass/volume%, or from 5 to 10 mass/volume%. In the present specification, the volume means a volume at 25°C and 1 atm.

The culture conditions may not be limited as long as the mutant strain of the present invention or the transformant of the present invention fermentatively produces a glycolipid under the conditions. The culture is preferably performed under aerobic conditions, and a general method such as aeration stirring culture or shake culture can be applied thereto. The culture temperature is preferably from 20 to 33°C, more preferably from 25 to 30°C, further more preferably from 28 to 30°C. The initial pH (30°C) of the medium is preferably from 2 to 7, more preferably from 3 to 6. The culture time is preferably about from 24 to 200 hours, more preferably from 50 to 200 hours.

In the culture, the mutant strain of the present invention or the transformant of the present invention may be cultured under cell proliferation conditions to fermentatively produce a glycolipid, or may be cultured in a dormant cell state, i.e., in a state where growth and proliferation are stopped, to fermentatively produce a glycolipid.

A method for recovering the glycolipid from the medium after culture is not particularly limited and can be performed in accordance with a recovery method known in the art. The glycolipid in the medium can be recovered or purified by using, for example, solvent extraction using ethyl acetate or butanol, fractional precipitation, liquid-liquid partition, column chromatography, and high-performance liquid chromatography alone or in appropriate combination.

The present specification further discloses a composition, a production method, application, or a method given below as an exemplary embodiment of the present invention. However, the present invention is not limited by these embodiments.

[1] A *Starmerella bombicola* mutant strain in which expression of a phosphoribosyl-glycinamide formyltransferase-like protein or a protein equivalent thereto is suppressed or the protein is inactivated.
[2] The mutant strain according to [1], wherein the phosphoribosyl-glycinamide formyltransferase-like protein is a polypeptide consisting of the amino acid sequence of SEQ ID NO: 2, and the protein equivalent to the phosphoribosyl-glycinamide formyltransferase-like protein is a polypeptide consisting of an amino acid sequence having at least 90% identity to the amino acid sequence of SEQ ID NO: 2.
[3] The mutant strain according to [1] or [2], wherein a gene encoding the phosphoribosyl-glycinamide formyltransferase-like protein or a gene equivalent thereto is deleted or inactivated.
[4] The mutant strain according to [3], wherein the gene encoding the phosphoribosyl-glycinamide formyltransferase-like protein is a gene consisting of the nucleotide sequence of SEQ ID NO: 1, and the gene equivalent to the gene of the phosphoribosyl-glycinamide formyltransferase-like protein is a gene consisting of a nucleotide sequence having at least 90% identity to the nucleotide sequence of SEQ ID NO: 1.
[5] The mutant strain according to any one of [1] to [4], wherein the mutant strain has adenine auxotrophy.
[6] The mutant strain according to any one of [1] to [5], wherein the mutant strain is a *Starmerella bombicola* T1 strain (accession No. NITE BP-04096).
[7] A method for producing a *Starmerella bombicola* mutant strain, comprising suppressing expression of or inactivating a phosphoribosyl-glycinamide formyltransferase-like protein or a protein equivalent thereto in *Starmerella bombicola.*
[8] The method according to [7], wherein the phosphoribosyl-glycinamide formyltransferase-like protein is a polypeptide consisting of the amino acid sequence of SEQ ID NO: 2, and the protein equivalent to the phosphoribosyl-glycinamide formyltransferase-like protein is a polypeptide consisting of an amino acid sequence having at least 90% identity to the amino acid sequence of SEQ ID NO: 2.
[9] The method according to [7] or [8], comprising deleting or inactivating a gene encoding the phosphoribosyl-glycinamide formyltransferase-like protein or a gene equivalent thereto.
[10] The method according to [9], wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.
[11] The method according to any one of [7] to [10], wherein the method is a method for producing an adenine auxotrophic *Starmerella bombicola* mutant strain.
[12] A method for transforming *Starmerella bombicola,* comprising using the mutant strain according to any one of [1] to [6] as a host.
[13] The method according to [12], further comprising introducing a gene encoding the phosphoribosyl-glycinamide formyltransferase-like protein or a gene equivalent thereto as an adenine auxotrophic selection marker into the host.
[14] The method according to [13], wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.
[15] The method according to any one of [12] to [14], further comprising selecting a transformant by using the presence or absence of adenine auxotrophy as an index.
[16] The method according to any one of [12] to [15], wherein the mutant strain is a mutant strain containing no foreign gene, preferably a *Starmerella bombicola* T1 strain, and a polynucleotide derived from the same species of microorganism as that of the host or a polynucleotide derived from a different species of microorganism from that of the host, provided that genetic exchange has been found to occur between the host and the microorganism in nature, preferably a polynucleotide derived from the same species of microorganism as that of the host, is introduced into the host.
[17] The method according to any one of [12] to [16], wherein a transformant capable of growing in an adenine-free medium is selected.
[18] A transformant obtained by the method according to any one of [12] to [17].
[19] The transformant according to [18], wherein expression of a polypeptide consisting of the amino acid sequence of SEQ ID NO: 23 or an amino acid sequence having at least 90% identity to the amino acid sequence of SEQ ID NO: 23 is suppressed or the protein is inactivated.
[20] The transformant according to [18] or [19], wherein a gene consisting of the nucleotide sequence of SEQ ID NO: 22 or a gene consisting of a nucleotide sequence having at least 90% identity to the nucleotide sequence of SEQ ID NO: 22 is deleted or inactivated.
[21] The transformant according to any one of [18] to [20], wherein expression of FAO1 or a protein equivalent thereto is expression or the FAO1 or the protein equivalent thereto is inactivated.
[22] The transformant according to [21], wherein the FAO1 is a polypeptide consisting of the amino acid sequence of SEQ ID NO: 35, and the protein equivalent to the FAO1 is a polypeptide consisting of an amino acid sequence having at least 90% identity to the amino acid sequence of SEQ ID NO: 35.
[23] The transformant according to any one of [18] to [22], wherein fao1 gene or a gene equivalent thereto is deleted or inactivated.
[24] The transformant according to [23], wherein the fao1 gene is a gene consisting of the nucleotide sequence of SEQ ID NO: 34, and the gene equivalent to the fao1 gene is a gene consisting of a nucleotide sequence having at least 90% identity to the nucleotide sequence of SEQ ID NO: 34.
[25] A method for producing a glycolipid, comprising culturing the mutant strain according to any one of [1] to [6] or the transformant according to any one of [18] to [24].
[26] The method according to [25], wherein the glycolipid is selected from the group consisting of sophorolipid, Bola sophorolipid, Bola sophoroside, alkyl sophoroside, alkyl glucoside, Bola glucoside, acidic glucolipid, and cellobiose lipid, and is preferably selected from the group consisting of sophorolipid, Bola sophoroside, and alkyl sophoroside.
[27] The method according to [25] or [26], wherein a medium for the culturing contains the following substrate:
   at least one substrate selected from the group consisting of C12 to C22 fatty acid and alkyl ester thereof, triacylglycerol, diacylglycerol, and monoacylglycerol containing C12 to C22 fatty acid or alkyl ester thereof, fat of oil containing C12 to C22 fatty acid or alkyl ester thereof, C12 to C22 alkane, C12 to C22 alkene, C12 to C22 alkyne, and C12 to C22 alcohol;
   at least one substrate selected from the group consisting of C12 to C18 fatty acid and alkyl ester thereof, triacylglycerol, diacylglycerol, and monoacylglycerol containing C12 to C18 fatty acid or alkyl ester thereof, fat or oil containing C12 to C18 fatty acid or alkyl ester thereof, C12 to C18 alkane, C12 to C18 alkene, C12 to C18 alkyne, and C12 to C18 alcohol; or
   at least one substrate selected from the group consisting of C12 to 18 fatty acid and alkyl ester thereof, and C12 to C18 alcohol.
[28] The method according to [27], wherein a content of the substrate in the medium is preferably 0.1 mass/volume% or more, more preferably 0.5 mass/volume% or more, further more preferably 1 mass/volume% or more, even more preferably 2 mass/volume% or more, even more preferably 3 mass/volume% or more, even more preferably 5 mass/volume% or more, and preferably 40 mass/volume% or less, more preferably 30 mass/volume% or less, further more preferably 25 mass/volume% or less, even more preferably 20 mass/volume% or less, even more preferably 15 mass/volume% or less, even more preferably 10 mass/volume% or less, or preferably from 0.1 to 40 mass/volume%, from 0.1 to 30 mass/volume%, from 0.1 to 25 mass/volume%, from 0.1 to 20 mass/volume%, from 0.1 to 15 mass/volume%, from 0.1 to 10 mass/volume%, from 0.5 to 40 mass/volume%, from 0.5 to 30 mass/volume%, from 0.5 to 25 mass/volume%, from 0.5 to 20 mass/volume%, from 0.5 to 15 mass/volume%, from 0.5 to 10 mass/volume%, from 1 to 40 mass/volume%, from 1 to 30 mass/volume%, from 1 to 25 mass/volume%, from 1 to 20 mass/volume%, from 1 to 15 mass/volume%, from 1 to 10 mass/volume%, from 2 to 40 mass/volume%, from 2 to 30 mass/volume%, from 2 to 25 mass/volume%, from 2 to 20 mass/volume%, from 2 to 15 mass/volume%, from 2 to 10 mass/volume%, from 3 to 40 mass/volume%, from 3 to 30 mass/volume%, from 3 to 25 mass/volume%, from 3 to 20 mass/volume%, from 3 to 15 mass/volume%, from 3 to 10 mass/volume%, from 5 to 40 mass/volume%, from 5 to 30 mass/volume%, from 5 to 25 mass/volume%, from 5 to 20 mass/volume%, from 5 to 15 mass/volume%, or from 5 to 10 mass/volume%.
[29] The method according to any one of [25] to [28], further comprising recovering the glycolipid from the medium after the culturing.
[30] An adenine auxotrophic selection marker consisting of a gene encoding a phosphoribosyl-glycinamide formyltransferase-like protein or a gene equivalent thereto, preferably an isolated gene encoding a phosphoribosyl-glycinamide formyltransferase-like protein or an isolated gene equivalent thereto.
[31] The adenine auxotrophic selection marker according to [30], wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.
[32] Use of a gene encoding a phosphoribosyl-glycinamide formyltransferase-like protein or a gene equivalent thereto, preferably an isolated gene encoding a phosphoribosyl-glycinamide formyltransferase-like protein or an isolated gene equivalent thereto as an adenine auxotrophic selection marker.
[33] The use according to [32], wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.
[34] A vector or a DNA fragment comprising a gene encoding a phosphoribosyl-glycinamide formyltransferase-like protein or a gene equivalent thereto.
[35] The vector or the DNA fragment according to [34], wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.

### Examples

Hereinafter, the present invention will be described in more detail with reference to Examples. However, the technical scope of the present invention is not limited by the following Examples.

### Example 1 Obtainment of Starmerella bombicola mutant strain

### (1) Obtainment of T1 strain

Semicontinuous culture aimed at glycolipid production was carried out using an NBRC10243 strain (ATCC22214 strain) as a type strain of *Starmerella bombicola.* One loopful of a glycerol stock of the NBRC10243 strain was inoculated to YPD agar medium (1 mass/volume% glucose, 1 mass/volume% yeast extracts, 1 mass/volume% tryptone, 1.5 mass/volume% agar) and statically cultured at 30°C. Subsequently, one loopful of grown cells was inoculated to a Sakaguchi flask supplemented with 75 mL of YPD medium (1 mass/volume% glucose, 1 mass/volume% yeast extracts, 1 mass/volume% tryptone) and shake-cultured at 120 rpm at 30°C for 42 hours. 24 mL of the seed culture solution mentioned above was added to 1.2 L of glycolipid production medium 1 (0.1 mass/volume% urea, 2 mass/volume% yeast extracts, 10 mass/volume% ethyl palmitate, 10 mass/volume% glucose) and stirring-cultured under a condition of 30°C in a 2 L jar fermenter. Then, aeration stirring and temperature control were stopped, and the culture solution was left standing for 1 hour, followed by the extraction of a glycolipid layer. 120 g of ethyl palmitate and 120 g of glucose were added to the remaining aqueous layer, and sterile water was added so as to adjust the volume of the mixture to 1.2 L. Then, aeration stirring and control at 30°C were resumed. The operation described above was repeated. The culture solution obtained by culture for 15 days was appropriately diluted, and a single colony was isolated in YPD agar medium. The isolated colony was cultured and evaluated in glycolipid production medium 1 again. A colony differing in properties during culture from a usual colony was designated as a *Starmerella bombicola* T1 strain. The T1 strain is a spontaneous mutant strain, and the genomic analysis of the T1 strain confirmed that a mutation was introduced into the gene described in SEQ ID NO: 1.

### (2) Confirmation of mutation in T1 strain

The presence or absence of amplification of the nucleotide sequence of SEQ ID NO: 1 was confirmed by PCR with the NBRC10243 strain, the T1 strain, or a single colony of each strain as a template using primers (SEQ ID NO: 3: ATGTCGATAGTTGTGCTGATTTCG, and SEQ ID NO: 4: CTAGATGCCCTTGTGAATGCGTG). PCR was performed as a control using primers (SEQ ID NO: 6: ATGCAGGACGATCAGTACGCTTTG, and SEQ ID NO: 7: TTAGGGACAGCGGCGGTGGAC) designed so as to amplify the nucleotide sequence of actin gene of SEQ ID NO: 5. As a result, the amplification of the nucleotide sequence of SEQ ID NO: 1 and the nucleotide sequence of SEQ ID NO: 5 were confirmed in the NBRC10243 strain, whereas the amplification of the nucleotide sequence of SEQ ID NO: 1 was not confirmed in the T1 strain although the amplification of the nucleotide sequence of SEQ ID NO: 5 was confirmed. From these results, the T1 strain had deletion of or mutation of the sequence of SEQ ID NO: 1.

### (3) Confirmation of adenine auxotrophy in T1 strain

The NBRC10243 strain and the T1 strain were separately inoculated to each of minimal agar medium (0.7 mass/volume% yeast nitrogen base (amino acid-free), 2 mass/volume% glucose, 1.5 mass/volume% agar) and minimal agar + adenine medium (0.03 mass/volume% adenine sulfate, 0.7 mass/volume% yeast nitrogen base (amino acid-free), 2 mass/volume% glucose, 1.5 mass/volume% agar) and statically cultured at 30°C. As a result, the NBRC10243 strain was observed to grow in both the media. By contrast, the T1 strain was observed to grow in the minimal agar + adenine medium, whereas its growth was not observed in the minimal agar medium (Fig. 1). Accordingly, the T1 strain was confirmed to have adenine auxotrophy.

### Example 2 Preparation of transformant and production of glycolipid - 1

### (1) Extraction of genomic DNA from NBRC10243 strain

Genomic DNA was extracted and purified from the NBRC10243 strain using Dr. GenTLE (for Yeast) High Recovery (Takara Bio Inc.) in accordance with the attached protocol.

### (2) Amplification of genomic DNA fragment containing nucleotide sequence of SEQ ID NO: 1

The enzyme used for PCR performed below was PrimeSTAR Max DNA Polymerase (Takara Bio Inc.) in all cases unless otherwise specified. A DNA fragment containing the nucleotide sequence of SEQ ID NO: 1 was amplified with the genomic DNA of the NBRC10243 strain as a template using primers (SEQ ID NO: 8: CCTCTTACCAATTGAACTATGGAAATTAATGAAGAATTGGGCTG, and SEQ ID NO: 9: TCGGACAACAATTCATCTGCAGACATAGCCGAAATTCTACTGTGAG) .

### (3) Amplification of 1 kbp upstream and 1 kbp downstream genomic DNA fragments of seq1 gene

Likewise, with the genomic DNA of the NBRC10243 strain as a template, 1 kbp upstream DNA fragment of seq1 gene was amplified using primers (SEQ ID NO: 10: GCCAAGCTTGCATGCTCCAATTTCTAAGGCGCAAGCGACGCTTCTAG, and SEQ ID NO: 11: TCAATTGGTAAGAGGGAACGCGTAG) and 1 kbp downstream NA fragment of seq1 gene was amplified using primers (SEQ ID NO: 12: TGAATTGTTGTCCGAATGCTCTGC, and SEQ ID NO: 13: AGAGTCGACCTGCAGCCCAACGCCTTGACAAGCTTTCCAAATAGAG) .

### (4) Amplification of vector fragment

A vector DNA fragment was amplified with pHSG298 (Takara Bio Inc.) as a template using primers (SEQ ID NO: 14: GCATGCAAGCTTGGCACTGGCCGTC, and SEQ ID NO: 15: CTGCAGGTCGACTCTAGAGGATCCCCG) .

### (5) Preparation of template plasmid pbJK8 of DNA fragment for introduction

Respective DNA fragments were purified from four PCR products described in the sections (2), (3), and (4) using NucleoSpin Gel and PCR Clean-up (Takara Bio Inc.) and then linked using In-Fusion HD cloning kit (Clontech Laboratories Inc.). Transformation into ECOS Competent E. Coli DH5α strain (Nippon Gene Co., Ltd.) was performed using the obtained plasmid solution, and the cell solution was applied to LB agar medium containing kanamycin and left standing overnight at 37°C. Colony PCR was performed with the obtained colony as a template using Sapphire Amp (Takara Bio Inc.) as an enzyme. The introduction of the DNA fragment of interest was confirmed using primers (SEQ ID NO: 16: CTCTTCGCTATTACGCCAGC, and SEQ ID NO: 17: CACTTTATGCTTCCGGCTCG). A transformant having the plasmid confirmed to have the introduced gene was inoculated to 2 mL of LB liquid medium containing kanamycin and cultured overnight at 37°C. The plasmid was purified from this culture solution using NucleoSpin Plasmid EasyPure (Takara Bio Inc.) to obtain a plasmid pbJK8 containing the DNA fragment in which the 1 kbp upstream fragment of the seq1 gene, the DNA fragment containing the nucleotide sequence of SEQ ID NO: 1, and the 1 kbp downstream fragment of the seq1 gene were linked.

### (6) Preparation of DNA fragment for introduction

The DNA fragment in which the 1 kbp upstream fragment of the seq1 gene, the DNA sequence including SEQ ID NO: 1, and the 1 kbp downstream fragment of the seq1 gene were linked was amplified with the plasmid pbJK8 as a template using primers (SEQ ID NO: 18: TCCAATTTCTAAGGCGCAAGCGAC, and SEQ ID NO: 19: CCCAACGCCTTGACAAGCTTTCC). The obtained PCR product was treated with DpnI (Takara Bio Inc.), and the DNA fragment was further purified using NucleoSpin Gel and PCR Clean-up (Takara Bio Inc.). The obtained DNA fragment was sequenced to confirm that this DNA fragment was entirely constituted by gene sequences present in the genome of the NBRC10243 strain.

### (7) Insertion of DNA fragment to T1 strain and preparation of T1Δseq1::ade strain

Transformation into the T1 strain by electroporation (Nepa Gene Co., Ltd.) was performed using the DNA fragment obtained in the section (6) in which the 1 kbp upstream fragment of the seq1 gene, the DNA sequence including SEQ ID NO: 1, and the 1 kbp downstream fragment of the seq1 gene were linked. The cell solution after transformation was applied to minimal agar medium and left standing at 30°C for 3 days. PCR was performed with the obtained colony as a template using primers (SEQ ID NO: 20: CATGATTGAGTGACGTACGATG, and SEQ ID NO: 21: CACAGTTCCATACAAGCCAG), and the amplified fragment was subjected to sequence analysis. The introduced DNA fragment was confirmed to be introduced on the genome. This strain was designated as a *Starmerella bombicola* T1Δseq1::ade strain. This strain had an insert of the DNA sequence containing the nucleotide sequence of SEQ ID NO: 1 on the genome so as to replace the seq1 gene. Deletion or inactivation of the seq1 gene (SEQ ID NO: 22) has been reported to improve the productivity of sophorolipid as compared with a parent strain (JP-B-6725506).

### (8) Confirmation of adenine auxotrophy in T1Δseq1::ade strain

The T1Δseq1::ade strain obtained in the section (7) and the host T1 strain were separately inoculated to minimal agar medium and statically cultured at 30°C. As a result, the growth of the T1 strain was not observed in the minimal agar medium. By contrast, the growth of the obtained T1Δseq1::ade strain was observed, confirming that adenine auxotrophy was canceled by the insertion of the nucleotide sequence of SEQ ID NO: 1.

From the results described above, it is evident that: a mutation introduced in the gene described in SEQ ID NO: 1 is responsible for the adenine auxotrophy of the T1 strain; and the gene described in SEQ ID NO: 1 is a gene involved in adenine auxotrophy.

### (9) Confirmation of glycolipid productivity of T1Δseq1::ade strain

The host T1 strain and the T1Δseq1::ade strain obtained in the section (8) were separately inoculated to glycolipid production medium 2 (0.1 mass/volume% urea, 2 mass/volume% yeast extracts, 5 mass/volume% oleic acid, 12.5 mass/volume% glucose) and shake-cultured under a condition of 30°C. 5 mL of the culture solution was recovered after culture for 72 hours, supplemented with 4 mL of hexane, and mixed by stirring for 5 seconds. After centrifugation for 5 minutes under conditions of 3 000 rpm and 25°C, a hexane fraction of a supernatant was removed. Subsequently, 6 mL of ethyl acetate was added to the remaining solution and mixed by stirring for 5 seconds. After centrifugation for 5 minutes under conditions of 3 000 rpm and 25°C, the whole amount of an ethyl acetate fraction was recovered. The recovered ethyl acetate fraction was volatilized by nitrogen gas blowing to deposit dissolved sophorolipid. The weight of the deposited sophorolipid was measured to calculate the concentration of the sophorolipid in the culture solution. The results are shown in Table 1. Produced sophorolipid was confirmed in each strain.

**[Table 1]**

| | Glycolipid concentration after culture for 72 hours (g/L) |
|---|---|
| T1 strain | 57.50 |
| T1Δseq1::ade strain | 75.00 |

### Example 3 Preparation of transformant and production of glycolipid - 2

### (1) Amplification of genomic DNA fragment containing nucleotide sequence of in SEQ ID NO: 1

The enzyme used for PCR performed below was PrimeSTAR Max DNA Polymerase (Takara Bio Inc.) in all cases unless otherwise specified. A DNA fragment containing the nucleotide sequence of SEQ ID NO: 1 was amplified with the genomic DNA of the NBRC10243 strain as a template using primers (SEQ ID NO: 24: TCCGGAATTGACACAACTATGGAAATTAATGAAGAATTGGGCTG, and SEQ ID NO: 25: TAAATCTAGATGATATCTGCAGACATAGCCGAAATTCTACTGTGAG).

### (2) Amplification of 1 kbp upstream and 1 kbp downstream genomic DNA fragments of fao1 gene

Likewise, with the genomic DNA of the NBRC10243 strain as a template, 1 kbp upstream DNA fragment of fao1 gene was amplified using primers (SEQ ID NO: 26: GCCAAGCTTGCATGCGAAGGGGCTCTCCGAAGTACATCACTG, and SEQ ID NO: 27: TGTGTCAATTCCGGAAAAACGACAGAAAAGTG) and 1 kbp downstream DNA fragment of fao1 gene was amplified using primers (SEQ ID NO: 28: TATCATCTAGATTTATATCACAAGTCACTATTTAC, and SEQ ID NO: 29: AGAGTCGACCTGCAGCTCAATGAACTGAGACAGCAGCTTGTCAC), respectively.

### (3) Amplification of vector fragment

A vector DNA fragment was amplified with pHSG298 (Takara Bio Inc.) as a template using primers (SEQ ID NO: 14: GCATGCAAGCTTGGCACTGGCCGTC, and SEQ ID NO: 15: CTGCAGGTCGACTCTAGAGGATCCCCG) .

### (4) Preparation of template plasmid pbJK8-2 of DNA fragment for introduction

Respective DNA fragments were purified from four PCR products described in the sections (1), (2), and (3) using NucleoSpin Gel and PCR Clean-up (Takara Bio Inc.) and then linked using In-Fusion HD cloning kit (Clontech Laboratories Inc.). transformation into ECOS Competent E. Coli DH5α strain (Nippon Gene Co., Ltd.) was performed using the obtained plasmid solution, and the cell solution was applied to LB agar medium containing kanamycin and left standing overnight at 37°C. Colony PCR was performed with the obtained colony as a template using Sapphire Amp (Takara Bio Inc.) as an enzyme. The introduction of the DNA fragment of interest was confirmed using primers (SEQ ID NO: 16: CTCTTCGCTATTACGCCAGC, and SEQ ID NO: 17: CACTTTATGCTTCCGGCTCG). A transformant having the plasmid confirmed to have the introduced gene was inoculated to 2 mL of LB liquid medium containing kanamycin and cultured overnight at 37°C. The plasmid was purified from this culture solution using NucleoSpin Plasmid EasyPure (Takara Bio Inc.) to obtain a plasmid pbJK8-2 containing the DNA fragment in which the 1 kbp upstream fragment of the fao1 gene, the DNA fragment containing the sequence of SEQ ID NO: 1, and the 1 kbp downstream fragment of the fao1 gene were linked.

### (5) Preparation of DNA fragment for introduction

The DNA fragment in which the 1 kbp upstream fragment of the fao1 gene, the DNA sequence including SEQ ID NO: 1, and the 1 kbp downstream fragment of the fao1 gene were linked was amplified with the plasmid pbJK8-2 as a template using primers (SEQ ID NO: 30: GAAGGGGCTCTCCGAAGTACATCACTG, and SEQ ID NO: 31: CTCAATGAACTGAGACAGCAGCTTGTCAC). The obtained PCR product was treated with DpnI (Takara Bio Inc.), and the DNA fragment was further purified using NucleoSpin Gel and PCR Clean-up (Takara Bio Inc.). The obtained DNA fragment was sequenced to confirm that this DNA fragment was entirely constituted by gene sequences present in the genome of the NBRC10243 strain.

### (6) Insertion of DNA fragment to T1 strain and preparation of T1Δfao1::ade strain

Transformation into the T1 strain by electroporation (Nepa Gene Co., Ltd.) was performed using the DNA fragment obtained in the section (5) in which the 1 kbp upstream fragment of the fao1 gene, the DNA sequence including SEQ ID NO: 1, and the 1 kbp downstream fragment of the fao1 gene were linked. The cell solution after transformation was applied to minimal agar medium and left standing at 30°C for 3 days. PCR was performed with the obtained colony as a template using primers (SEQ ID NO: 32: CTAAGGCCAGTACAAGAGATC, and SEQ ID NO: 33: CTAAAGCTCGAATTAATAGAGACACG), and the amplified fragment was subjected to sequence analysis. The introduced DNA fragment was confirmed to be introduced on the genome. This strain was designated as a *Starmerella bombicola* T1Δfao1::ade strain. This strain had an insert of the DNA sequence containing the nucleotide sequence of SEQ ID NO: 1 on the genome so as to replace the fao1 gene. Deletion or inactivation of the fao1 gene (SEQ ID NO: 34) has been reported to mainly produce Bola sophoroside and alkyl polyglycoside when alcohols are used as substrates (e.g., Appl Microbiol Biotechnol. 2016 Nov; 100 (22): 9519-9528).

### (7) Confirmation of adenine auxotrophy in T1Δfao1::ade strain

The T1Δfao1::ade strain obtained in the section (6) and the host T1 strain were separately inoculated to minimal agar medium and statically cultured at 30°C. As a result, the growth of the T1 strain was not observed in the minimal agar medium. By contrast, the growth of the obtained T1Δfao1::ade strain was observed, confirming that adenine auxotrophy was canceled by the insertion of the nucleotide sequence of SEQ ID NO: 1.

### (8) Confirmation of glycolipid productivity of T1Δfao1::ade strain

The T1Δfao1::ade strain obtained in the section (7) was inoculated to glycolipid production medium 3 (0.5 mass/volume% trisodium citrate dihydrate, 0.4 mass/volume% yeast extracts, 0.15 mass/volume% ammonium chloride, 0.07 mass/volume% magnesium sulfate heptahydrate, 0.05 mass/volume% sodium chloride, 0.027 mass/volume% calcium chloride dihydrate, 0.1 mass/volume% potassium dihydrogen phosphate, 0.016 mass/volume% dipotassium hydrogen phosphate, 15 mass/volume% glucose) and shake-cultured under a condition of 30°C. Oleyl alcohol (2.6 mass/volume%) was added thereto as a substrate after culture for 1 day. 3 mL of the culture solution was recovered after culture for 4 days, supplemented with 3 mL of hexane, and mixed by stirring for 5 seconds. After centrifugation for 5 minutes under conditions of 3 000 rpm at 25°C, a hexane fraction of a supernatant was removed. Subsequently, 3 mL of butanol was added to the remaining solution and mixed by stirring for 5 seconds. After centrifugation for 5 minutes under conditions of 3 000 rpm at 25°C, the whole amount of a butanol fraction was recovered. 3 mL of butanol was further added to the remaining solution. The same operation as above was repeated, and the whole amount of a butanol fraction was recovered. The recovered butanol fraction was volatilized by nitrogen gas blowing, and the weight of deposits from the dissolved matter was measured and defined as the concentration of Bola sophoroside in the culture solution. As a result, Bola sophoroside produced at 37.7 g/L was confirmed in the T1Δfao1::ade strain.

## Claims

1. A *Starmerella bombicola* mutant strain in which expression of a phosphoribosyl-glycinamide formyltransferase-like protein or a protein equivalent thereto is suppressed or the protein is inactivated.

2. The mutant strain according to claim 1, wherein the phosphoribosyl-glycinamide formyltransferase-like protein is a polypeptide consisting of the amino acid sequence of SEQ ID NO: 2, and the protein equivalent to the phosphoribosyl-glycinamide formyltransferase-like protein is a polypeptide consisting of an amino acid sequence having at least 90% identity to the amino acid sequence of SEQ ID NO: 2.

3. The mutant strain according to claim 1 or 2, wherein a gene encoding the phosphoribosyl-glycinamide formyltransferase-like protein or a gene equivalent thereto is deleted or inactivated.

4. The mutant strain according to claim 3, wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.

5. The mutant strain according to any one of claims 1 to 4, wherein the mutant strain has adenine auxotrophy.

6. The mutant strain according to any one of claims 1 to 5, wherein the mutant strain is a *Starmerella bombicola* T1 strain (accession No. NITE BP-04096).

7. A method for producing a *Starmerella bombicola* mutant strain, comprising suppressing expression of or inactivating a phosphoribosyl-glycinamide formyltransferase-like protein or a protein equivalent thereto in *Starmerella bombicola.*

8. The method according to claim 7, wherein the phosphoribosyl-glycinamide formyltransferase-like protein is a polypeptide consisting of the amino acid sequence of SEQ ID NO: 2, and the protein equivalent to the phosphoribosyl-glycinamide formyltransferase-like protein is a polypeptide consisting of an amino acid sequence having at least 90% identity to the amino acid sequence of SEQ ID NO: 2.

9. The method according to claim 7 or 8, comprising deleting or inactivating a gene encoding the phosphoribosyl-glycinamide formyltransferase-like protein or a gene equivalent thereto.

10. The method according to claim 9, wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequenceof SEQ ID NO: 1.

11. The method according to any one of claims 7 to 10, wherein the method is a method for producing an adenine auxotrophic *Starmerella bombicola* mutant strain.

12. A method for transforming *Starmerella bombicola,* comprising using the mutant strain according to any one of claims 1 to 6 as a host.

13. The method according to claim 12, further comprising introducing a gene encoding the phosphoribosyl-glycinamide formyltransferase-like protein or a gene equivalent thereto as an adenine auxotrophic selection marker into the host.

14. The method according to claim 13, wherein the gene encoding the phosphoribosyl-glycinamide formyltransferase-like protein is a gene consisting of the nucleotide sequence of SEQ ID NO: 1, and the gene equivalent to the gene encoding the phosphoribosyl-glycinamide formyltransferase-like protein is a gene consisting of a nucleotide sequence having at least 90% identity to the nucleotide sequence of SEQ ID NO: 1.

15. The method according to claim 13 or 14, further comprising selecting a transformant by using the presence or absence of adenine auxotrophy as an index.

16. A transformant obtained by the method according to any one of claims 12 to 15.

17. The transformant according to claim 16, wherein expression of a polypeptide consisting of the amino acid sequence of SEQ ID NO: 23 or an amino acid sequence having at least 90% identity to the amino acid sequence of SEQ ID NO: 23 is suppressed or the protein is inactivated.

18. The transformant according to claim 16 or 17, wherein expression of FAO1 or a protein equivalent thereto is suppressed or FAO1 or a protein equivalent thereto is inactivated.

19. A method for producing a glycolipid, comprising culturing the mutant strain according to any one of claims 1 to 6 or the transformant according to any one of claims 16 to 18.

20. The method according to claim 19, wherein the glycolipid is selected from the group consisting of sophorolipid, Bola sophorolipid, Bola sophoroside, alkyl sophoroside, alkyl glucoside, Bola glucoside, acidic glucolipid, and cellobiose lipid.

21. An adenine auxotrophic selection marker consisting of a gene encoding a phosphoribosyl-glycinamide formyltransferase-like protein or a gene equivalent thereto.

22. The adenine auxotrophic selection marker according to claim 21, wherein the gene encoding a phosphoribosyl-glycinamide formyltransferase-like protein is a gene consisting of the nucleotide sequence of SEQ ID NO: 1, and the gene equivalent to the gene encoding a phosphoribosyl-glycinamide formyltransferase-like protein is a gene consisting of a nucleotide sequence having at least 90% identity to the nucleotide sequence of SEQ ID NO:1.

23. A vector or a DNA fragment comprising a gene encoding a phosphoribosyl-glycinamide formyltransferase-like protein or a gene equivalent thereto.
